(19)

Europäisches
Patentamt
European
Patent Office
Office européen
des brevets

(11)  EP 3 415 088 A1

(12)  EUROPEAN PATENT APPLICATION

(43) Date of publication:
19.12.2018  Bulletin 2018/51

(51) Int Cl.:
A61B 5/0484 (2006.01)  A61B 5/00 (2006.01)

(21) Application number: 17305703.5

(22) Date of filing: 12.06.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: RYTHM
75009 Paris (FR)

(72) Inventors:
• MERCIER, Hugo
75010 PARIS (FR)

• SOULET DE BRUGIERE, Quentin
75001 PARIS (FR)
• KALIFA, Jérôme
92160 ANTONY (FR)
• GALTIER, Mathieu
75013 PARIS (FR)
• PINEAUD, Clémence
75014 PARIS (FR)
• DEHAENE, David
75002 PARIS (FR)

(74) Representative: Cabinet Plasseraud
66, rue de la Chaussée d'Antin
75440 Paris Cedex 09 (FR)

(54)  **METHOD, SYSTEM AND COMPUTER PROGRAM TO MEASURE IN REAL-TIME THE PHASE OF A PSEUDO-PERIODIC PHYSIOLOGICAL SIGNAL OF A USER**

(57)    According to the method to measure in real-time the phase of a pseudo-periodic physiological signal of a user:
- one provides a physiological signal (S) of the user;
- a trigonometric function determination module (14) of a processor determines parameters of a trigonometric function, which minimize a distance between said physiological signal (S) and said trigonometric function on the time interval $]t_a ; t_b[$ ;
- a phase determination module (13) of a processor determines the phase ($f_{ps}$) of the physiological signal at time $t_b$ based on the phase ($f_{bf}$) of the trigonometric function at time $t_b$.

FIG. 1

## Description

The invention relates to methods, systems and computer programs to measure the phase of a pseudo-periodic physiological signal of a user.

More precisely, the invention relates to methods to measure the phase of a pseudo-periodic physiological signal of a user. A physiological signal of a user can be measured in different ways. One typical way is to measure a physiological signal of a user using a suitable sensor, as part of a medical examination procedure. This is generally performed using specific material by professional personal (such as nurses or doctors) which are experienced with this material.

There is a recent trend for measuring physiological signals of someone by untrained personal, very often by the person itself. This would enable to obtain signal measurements over a longer period of time than what is typically done during a medical examination procedure. However, the measurement conditions are adverse because the measurement will be set by the untrained user and may be subjected to aggressive every-day life conditions.

Such physiological signals are typically pseudo-periodic. This reflects the cycles of human living. By "pseudo-periodic", it is meant that the signal is not exactly periodic according to the strict mathematical definition, but roughly follows a shape similar to that of a trigonometric function. Taking as an example the breathing cycle, it comprises an inspiration phase and an expiration phase which together toughly form a period of a trigonometric signal. For one cycle, the period is the time duration between the start of two successive inspirations. Due to biological processes, the period is not exactly constant over time, but varies with time. The phase is defined as the instantaneous position within a given cycle. Another typical example of a pseudo-periodic physiological signal which can be used within the frame of the invention is an electro-encephalogram system.

It is possible, based on a previously measured signal, to determine the exact period of a full oscillation, and, a posteriori, the exact phase within the period at any given time within the period. However, this requires, at said given time, to know the shape of the signal after this given time. In other words, it is not possible to determine the instantaneous phase of the signal.

One strives to determine the instantaneous phase of a pseudo-periodic biolological signal in real time. This would be useful to successfully drive a process based on the instantaneous phase of the biological signal.

According to a first object, the invention relates to a method to measure in real-time the phase of a pseudo-periodic physiological signal of a user, wherein :

- one provides a physiological signal of the user measured continuously during a time interval $]t_a$ ; $t_b[$ extending between times $t_a$ and $t_b$, and of duration Dt, where the physiological signal does not extend after time $t_b$ ;
- a trigonometric function determination module of a processor determines parameters of a trigonometric function, which minimize a distance between said physiological signal and said trigonometric function on the time interval $]t_a$ ; $t_b[$, said trigonometric function being defined by at least one pulsation and a phase as said parameters ;
- a phase determination module of a processor determines the phase of the physiological signal at time $t_b$ based on the phase ($f_{bf}$) of the trigonometric function at time $t_b$.

Thanks to these features, the instantaneous phase of the signal can be measured in real time.

According to some embodiments, one may use one or more of the following features:

- the trigonometric function determination module comprises a distance determination module which affects a greater weight to points of the physiological signal closer to time $t_b$ than far away from time $t_b$.;

- the phase determination module is part of a signal characterization module which also comprises a pulsation determination module which determines the pulsation of the physiological signal on time interval $]t_a$ ; $t_b[$ based on the pulsation of the trigonometric function in the time interval $]t_a$ ; $t_b[$;

- the pseudo-periodic physiological signal has a frequency between 0.1 and 2 Hz;

- the duration Dt is at least 100% of an average period of the pseudo-periodic physiological signal, at least 150% of that period, or at least 200% of that period;

- the physiological signal is an electro-encephalogram signal;

- the physiological signal is a plethysmography signal;

the method further comprises:

a. one provides a physiological signal of the user measured continuously during a time interval $]t_c ; t_d[$ extending between times $t_c$ and $t_d$, where time $t_b$ is between time $t_c$ and time $t_d$, where the physiological signal does not extend after $t_d$ ;

b. said trigonometric function determination module of a processor determines the parameters of a trigonometric function, which minimize a distance between said physiological signal and said trigonometric function on the time interval $]tc ; td[$, said trigonometric function being defined by at least one pulsation and a phase ;

c. said phase determination module of a processor determines the phase of the physiological signal at time $t_d$ based on the phase of the trigonometric function at time $t_d$;

- the time interval $]t_c ; t_d[$ is of duration Dt;

- wherein the phase determination module is executed at a frequency at least over 25 Hz;

- the phase determination module of a processor determines the phase of the physiological signal at time $t_d$ based at least on the phase of the trigonometric function at time $t_b$;

the method further comprises measuring said physiological signal with a sensor;
the method further comprises emitting an acoustic stimulation signal based on the phase determined by the phase determination module.

According to another aspect, the invention relates to a computer program comprising instructions adapted to perform the steps of the above methods wherein the computer program is run on a processor.

According to another aspect, the invention relates to a system to measure in real-time the phase of a pseudo-periodic physiological signal of a user, comprising, a physiological signal of the user being provided, which was measured continuously during a time interval $]t_a ; t_b[$ extending between times $t_a$ and $t_b$, and of duration Dt, where the physiological signal does not extend after $t_b$ :

d. a trigonometric function determination module of a processor adapted to determine the parameters of a trigonometric function, which minimize a distance between said physiological signal and said trigonometric function on the time interval $]t_a ; t_b[$, said trigonometric function being defined by at least one pulsation and a phase ;

e. a phase determination module of a processor adapted to determine the phase of the physiological signal at time $t_b$ based on the phase of the trigonometric function at time $t_b$.

According to some embodiments, the system may comprise one or more features adapted to perform the above methods.

The list of drawings hereby follows:

Figure 1 is a schematic view of a self-contained device for stimulating brain waves that is worn on the head of a person, according to one embodiment of the invention,

Figure 2 is a detail perspective view of a self-contained device for stimulating brain waves according to one embodiment of the invention, where the device comprises in particular first and second acoustic transducers respectively adapted to emit acoustic signals respectively stimulating a right inner ear and a left inner ear of the person,

Figure 3 is a block diagram of the device of Figure 2, illustrating the elements of the device and the functional links between these elements,

Figure 4 is a schematic view of the electronics,

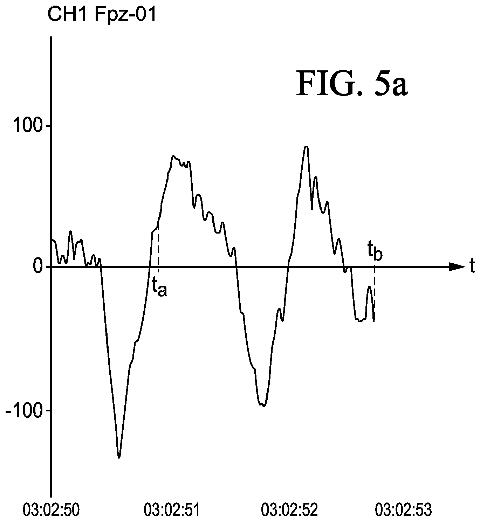

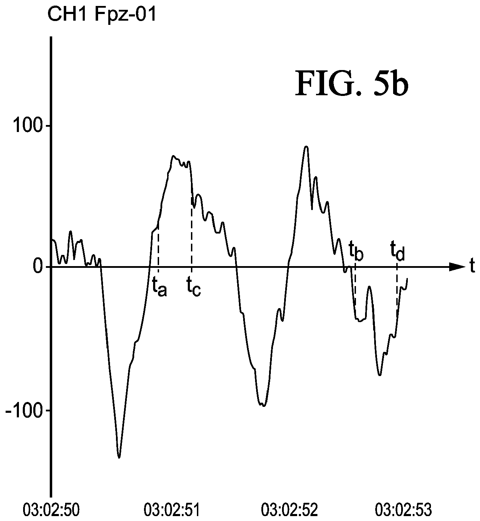

Figures 5a and 5b are graphs showing a electroencephalogram at times $t_b$ and $t_d$, respectively,

Figure 6 is a view similar to figure 5 showing a full plethysmogram.

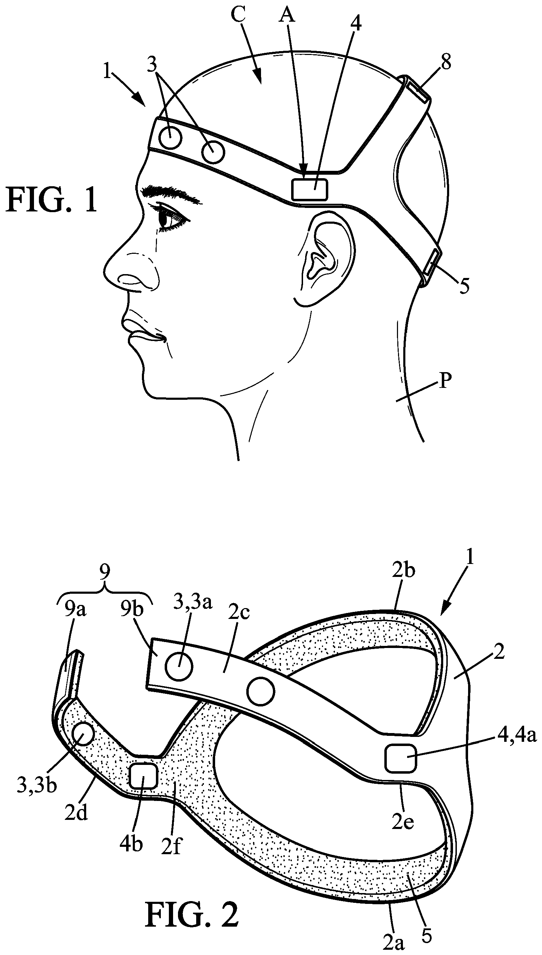

On the figures, identical or similar elements are designated using the same reference sign.
Thereafter, one or more embodiments of the invention will be described.
Referring firstly to Figures 1 and 2, a first object of the invention is a device 1 for stimulating brain waves.
The device 1 is adapted to be worn by a person P, in particular during the person's sleep period.
The device is adapted in particular to be worn on the head of the person P.
To this end, the device 1 comprises a supporting member 2. The supporting member 2 is adapted to surround the head of the person P at least partially so as to be held thereon. In one embodiment of the invention illustrated in Figure 1, the supporting member 2 is particularly adapted to surround at least a portion of a circumference of the head of the person P, in particular surrounding at least half of a circumference of the head of the person P, or even entirely surrounding a diameter of the head of the person P.

In the embodiment illustrated in Figure 1, the supporting member 2 has several arms 2a, 2b, 2c, 2d. The supporting member comprises in particular four arms interconnected at arm connection points 2e, 2f. The arms 2a, 2b, 2c, 2d surround different portions of the head of the person P so as ensure stable retention and a precise positioning of the device 1 on the person P.

For example, a first arm 2a surrounds a back of the head, and a second arm 2b surrounds the top of the head. The first and second arms 2a, 2b are respectively connected at their respective ends at a left lateral arm connection point 2e and a right lateral arm connection point 2f, respectively located near the left and right temples of the person P. Finally, the third and fourth arms 2c, 2d respectively extend from the left lateral 2e and right lateral 2f arm connection points, towards the front of the person P.

The device 1 further comprises a plurality of electrodes 3, at least one acoustic transducer 4, and embedded conditioning and control electronics 5.

The electrodes 3, acoustic transducer 4, and electronics 5 are operatively connected to each other. Thus, the embedded conditioning and control electronics 5 are particularly suitable for controlling and for receiving information from the plurality of electrodes 3, and are also able to command and control the emission of an acoustic signal A by the acoustic transducer 4.

To this end, the electrodes 3, the acoustic transducer 4, and the electronics 5 are mounted on the supporting member 2. In this manner the electrodes 3, the acoustic transducer 4, and the electronics 5 are close to each other so that communication between these members 3, 4, 5 is particularly fast and high speed. In the example of Figure 1, the electrodes are mounted on the third and fourth arms 2c, 2d, the electronics 5 are mounted on the first arm 2a, and two acoustic transducers 4 are respectively mounted near the left lateral 2e and right lateral 2f arm connection points. Other arrangements of the components of the device 1 are possible.

This allows implementing an operation of stimulating the brain waves of a person P in soft real-time.

Thus, in particular, the electronics 5 are capable, in soft real-time, of receiving a measurement signal S from the plurality of electrodes 3 and controlling the emission by the acoustic transducer of an acoustic signal A synchronized with a predefined temporal pattern T of a brain wave of the person P.

"Synchronized with a predefined temporal pattern of a brain wave" is understood to mean that the acoustic signal emitted by the device is temporally synchronized with a brain wave of the person. More precisely, it means that the acoustic signal emitted by the device is temporally synchronized with an instantaneous phase of a brain wave of the person as detailed below.

"Soft real-time" is understood to mean an implementation of the stimulation operation such that the time constraints on this operation, in particular the duration of the operation or the frequency at which it is repeated, are satisfied on the average over a predefined total implementation duration, for example a few hours. It is understood that the implementation of said operation may at certain times exceed said time constraints as long as the average operation of the device 1 and the average implementation of the method satisfies these constraints over the predefined total implementation duration. Time limits may be predefined, beyond which the implementation of the stimulation operation is to be stopped or paused.

To enable such an implementation in soft real-time, a maximum distance between the electrodes 3, the acoustic transducer 4, and the electronics 5 may be less than approximately one meter and preferably less than a few decimeters, enabling them to be connected through a wire embedded in the wearable. In this manner, sufficiently rapid communication between the elements of the device 1 can be guaranteed.

The electrodes 3, the acoustic transducer 4, and the electronics 5 may for example be housed in cavities of the supporting member 2, snapped onto the supporting member 2, or attached to the supporting member 2 for example by gluing, screwing, or other suitable means of attachment. In one embodiment of the invention, the electrodes 3, the acoustic transducer 4, and the electronics 5 may be detachably mounted on the supporting member 2.

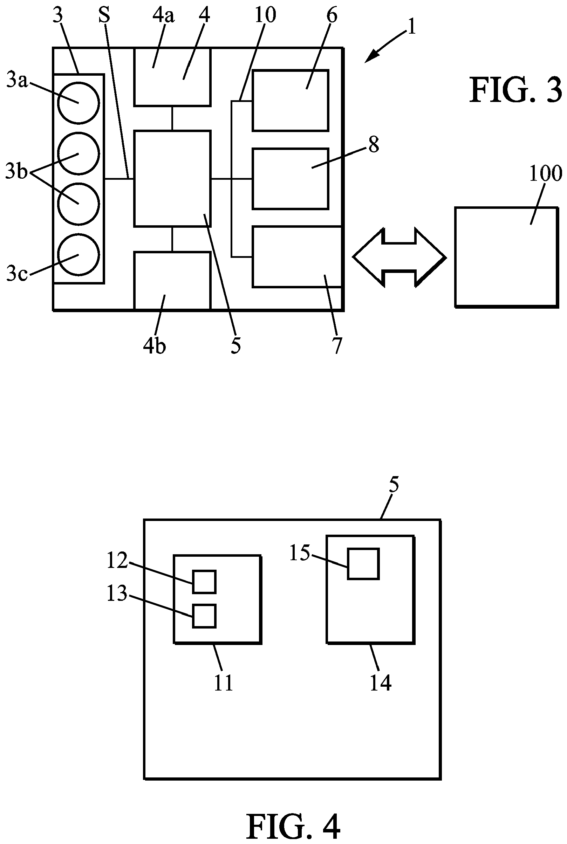

Referring now to Figure 3 as well, in one advantageous embodiment of the invention, the embedded conditioning and control electronics 5 are operatively connected to the electrodes 3 and to the acoustic transducer 4 by means of wire connections 10. In this manner, exposure of the person P to electromagnetic radiation is reduced.

The acoustic transducer 4 is adapted to emit an acoustic signal A stimulating at least one inner ear of the person P.

In a first embodiment illustrated in particular in Figures 1 and 2, the acoustic transducer 4 is an osteophonic device stimulating the inner ear of the person P by bone conduction.

This osteophonic device 4 may for example be adapted for placement near the ear, for example above it as illustrated in Figure 1, in particular on a region of skin covering a cranial bone.

In a second embodiment, the acoustic transducer 4 is a speaker stimulating the inner ear of the person P via an ear canal leading to said inner ear.

This speaker may be placed outside the ear of the person P or in the ear canal.

The acoustic signal A is a modulated signal that at least partially lies within a frequency range audible to a person P, for example the range of 20Hz to 30kHz.

The electrodes 3 are adapted to be in contact with the person P, and in particular with the skin of the person P, in order to capture at least one measurement signal S representative of a physiologic electrical signal E of the person P.

The physiological electrical signal E may in particular be an electroencephalogram (EEG), electro-myogram (EMG), electrooculogram (EOG), photoplethysmogram, pulse-oxygram and accelerometer or any other biosignal measurable in a person P, in particular those with a waveform close to a sinus function.

In particular, the physiological electrical signal E advantageously is an electroencephalogram (EEG) of the person P.

To this end, in one embodiment of the invention, the device 1 comprises at least two electrodes 3 of which at least one is a reference electrode 3a and at least one is an EEG measurement electrode 3b.

The device 1 may further comprise a ground electrode 3c.

In one particular embodiment, the device 1 comprises at least three EEG measurement electrodes 3, so as to capture physiological electrical signals E comprising at least three electroencephalogram measurement channels.

The EEG measurement electrodes 3 are for example arranged on the surface of the scalp of the person P.

In other embodiments, the device 1 may further comprise an EMG measurement electrode, and possibly an EOG measurement electrode.

The measurement electrodes 3 may be reusable electrodes or disposable electrodes. Advantageously, the measurement electrodes 3 are reusable electrodes in order to simplify the everyday use of the device.

The measurement electrodes 3 may be dry electrodes or electrodes coated with contact gel. The electrodes 3 may also be textile or silicone electrodes.

In one embodiment of the invention, the measurement electrodes 3 are active electrodes adapted to preprocess the measurement signal S, for example to perform at least one of the following preprocessing operations:

- frequency filtering, for example frequency filtering of the measurement signal S within a range of temporal frequencies of interest, for example a frequency range within 0.3 Hz to 100 Hz,
- amplification, for example amplification of the measurement signal S by a factor ranging from $10^3$ to $10^6$, and/or
- sampling the measurement signal S by means of an analog-to-digital converter adapted, for example, to sample the measurement signal S at a sampling rate of several hundred Hertz, for example 256 Hz or 512 Hz.

Such preprocessing of the measurement signal S may for example be implemented by an analog module of the measurement electrode 3 or by an analog module located near the measurement electrode 3.

The embedded conditioning and control electronics 5 receive the measurement signals S from the electrodes 3, possibly preprocessed as detailed above.

Alternatively, one may use other kinds of sensors to measure physiological signals of the user. These may include one or more of a pulse-oxymeter and/or inertial sensors. Physiological signals may thus include a movement signal, such as a respiratory movement signal, obtained from an accelerometer, or a signal representative of respiration, such as obtained from a pulse-oxymeter and/or a signal representative of the cardiac rythm. Other considered signals may include body temperature, body sound and/or body vibrations signals.

If the measurement signals S received by the electronics 5 are not preprocessed, the electronics 5 may apply one and/or more preprocessing operations as detailed above.

The embedded conditioning electronics 5 include one or more microchips, for example at least one microprocessor.

As detailed above, the embedded conditioning and control electronics 5 are adapted to implement an operation of stimulating brain waves of the person P, an operation which will later be described in more detail.

Said means of the embedded conditioning and control electronics 5 are for example microchips, microprocessors, and/or electronic memories, where appropriate mounted and interconnected on flexible or rigid printed circuit boards and operatively connected to the electrodes 3 and to the transducer 4 via wired connections 10.

The device 1 may further comprise a memory 6 as illustrated in Figure 3. The memory 6 is adapted to be mounted on the supporting member 2, for example as described above for the electrodes 3, the acoustic transducer 4, and the electronics 5. The memory 6 may be permanently mounted on the supporting member 1 or may be a removable module, for example a memory card such as an SD card (acronym for "Secure Digital").

The memory 6 is operatively connected to the electronics 5. The memory 6 may be controlled by the embedded conditioning and control electronics 5 so as to store the measurement signals S.

In one advantageous embodiment of the invention, the memory 6 is capable of storing measurement signals S for a duration of several hours, for example at least eight hours so as to cover an average sleep period of a person P.

The device 1 may further comprise a communication module 7 for communicating with an external server 100.

The communication module 7 may be mounted on the supporting member 1 as described above for the electrodes 3, the acoustic transducer 4, and electronics 5. The communication module 7 may be controlled by the embedded conditioning and control electronics 5.

The electronics 5 may in particular be adapted to control the communication module 7 to transfer the measurement signals S stored in memory 6 to an external server 100. The transfer operation may be implemented after a sleep period of the person P.

The communication module 7 may advantageously be a wireless communication module, for example a module implementing a protocol such as Bluetooth or Wi-Fi.

In this manner, when the P person is in a sleep period, he or she is not disturbed by cables, in particular if it is necessary to transmit data during the sleep period.

The device 1 may also comprise a battery 8. The battery 8 may be mounted on the supporting member 1 as described above for the electrodes 3, the acoustic transducer 4, and the electronics 5. The battery 8 may be capable of supplying power to the plurality of electrodes 3, the acoustic transducer 4, and the electronics 5, and where appropriate the memory 6 and the communication module 7. The battery 8 is preferably adapted to supply power for several hours without recharging, more preferably for at least eight hours so as to cover an average sleep period of a person P.

The device 1 can thus operate autonomously during a sleep period of the person P. In this manner in particular, the device 1 is self-contained and adapted to implement one or more operations of stimulating slow brain waves without communicating with an external server 100, in particular without communicating with an external server 100 for several minutes, more preferably several hours, more preferably at least eight hours. This reduces the exposure of the person P to electromagnetic radiation. In particular, the device 1 may also be used to assist the person with falling asleep.

"Self-contained" is thus understood to mean that the device can operate for an extended period of several minutes, preferably several hours, in particular at least eight hours, without needing to be recharged with electrical energy, communicate with external elements such as an external server, or be structurally connected to an external device such as a securing member such as an arm or a bracket.

In this manner the device is suitable for use in the everyday life of a person P without imposing particular constraints.

Furthermore, the supporting member 2 advantageously comprises a device 9 for adjusting to the diameter of the head of the person P. This allows adjusting device 1 to the person P and therefore enables particularly good contact between the electrodes 3 and the skin of the person P.

The adjustment device 9 allows changing a dimension of the supporting member 2 according to a diameter of the head of a person P, to allow fine-tuned adjustment to said diameter.

In one embodiment illustrated in particular in Figure 1, the adjustment device 9 comprises at least two parts 9a, 9b that are movable with respect to one another. The parts 9a, 9b may be rigid or semi-rigid. In the example of Figure 1, the parts 9a and 9b are respectively the ends of the third and fourth arms 2c, 2d of the supporting member 2. The device 1 can be adjusted to and remain in place on the head of the person P.

In a variant of this embodiment, the adjustment device 9 may also include a lock adapted to prevent or allow a relative movement of said two parts 9a, 9b. The lock may be an integral part of one of parts 9a, 9b or may be an element independent of the two parts 9a, 9b.

In another embodiment of the invention, the adjustment device 9 is a soft and flexible portion of the supporting member 2. This portion may be a portion of fabric or elastomer, for example of stretch fabric.

The physiological signal is a pseudo-periodic signal. For the example of slow brain waves, as shown on Fig. 4a, the frequency of such signal is of the order of 1 Hz. It should be reminded that the period is defined as the inverse of the frequency. It should be reminded that the pulsation is defined as 2 times pi times the frequency, where pi is the abstract number approximately equal to 3,14. At a given time tb, the measured signal is known at any time before tb. It can be made use of the signal over an interval $]ta; tb[$, where ta is chosen sufficiently far away from tb. For example, the duration Dt=tb-ta is at least 100% of an average period of the pseudo-periodic physiological signal, at least 150% of that average period, or at least 200% of that average period. It may even be more than two times the average period. At the given time tb, the future signal to be measured (for times after tb) is still unknown.

The electronics 5 comprise a signal characterization module 11. The signal characterization module 11 may comprise a pulsation determination module 12 and a phase determination module 13.

The pulsation determination module 12 determines the pulsation $w_{ps}$ of the physiological signal during time interval $]ta ; tb[$. This may for example be based on a trigonometric base function having a pulsation $w_{bf}$. According to such an example, the pulsation $w_{ps}$ of the physiological signal can thus be estimated as equal to $w_{bf}$.

The phase determination module 13 determines the phase $f_{ps}$ of the physiological signal at time $t_b$. This may for example be based on a trigonometric base function having a phase $f_{bf}$ at time $t_1$. According to such an example, the phase $f_{ps}$ of the physiological signal at $t_b$ can thus be estimated as equal to $f_{bf}$.

According to some embodiments, the pulsation determination module 12 and the phase determination module 13 can be inter-mixed, so that the pulsation $w_{ps}$ of the physiological signal during time interval $]t0 ; t1[$ and the phase $f_{ps}$

of the physiological signal at time $t_b$ can be determined together.

According to a first step, one provides with signal data y = [y1, ..., yn] on time window frame t = [t1, ..., tn] (t1=ta ; tn=tb). This signal can be modelised using a trigonometric function, such as a cosinus function:

One defines the value y' at point k of the base function as follows: y'[k] = Y0 cos(w * t[k] + phi[k]), where w is the pulsation of the base function, phi[k] is the phase of the base function at point k, and Y0 its amplitude.

Alternately, y'[k] can be written as y'[n] = A cos(w * t[k]) + B sin(w * t[k]), where A and B are unknown parameters.

One defines a plurality of candidates $w_j$ for the pulsation of the signal. In particular, the candidates are selected within a pre-determined pulsation range. The pre-determined pulsation range can be pre-determined in the system, and will depend of the nature of the physiological signal. For example, the pre-determined pulsation range can be a maximal pulsation range designed to cover every likely possibilities of pulsation for the physiological signal under study. For example, the maximal pulsation range will be between 50% and 150% of the average pulsation for this kind of physiological signal.

If the pulsation determination module has already been used recently (in particular since the present process is supposed to be used in real-time at a high frequency), the pre-determined pulsation range can be determined based on the estimated pulsation at the previous use. This is likely to enable a much narrower pre-determined pulsation range. For example, the pre-determined pulsation range could be between 90% and 110% of the estimated pulsation at the previous use of the pulsation determination module. This is for example the case when the phase of the input signal is needed at any time. This is for example the case when a stimulation is performed based on the instantaneous phase of the input signal, such as, for example, when the stimulation is performed based on the respiratory rhythm of the user.

The number of candidates $w_j$ is for example 10 to 50.. These can be selected uniformly, under a pre-determined repartition key (for example providing more candidates close to the middle of the pre-determined pulsation range than on the edges), or randomly in the pre-determined pulsation range.

A trigonometric function determination module 14 determines the trigonometric function y' which best approximates the signal. Determining the pulsation w for interval ]ta; tb[ and the phase at time tb can be performed by minimizing a distance between the functions y and y' over interval ]ta; tb[. A distance determination module 15 can be used to determine this distance.

The distance can be any suitable distance.

In particular, the distance determination module may affect a greater weight to points of the physiological signal closer to tb than far away from tb.

For example, for a given pulsation candidate $w_j$, the distance determination module may solve the following least-square problem:

$$e[theta] = 1 / N \; sum_k((y[k] - y'[k])^2 * lambda[k])$$

where lambda is a forgetting factor vector, theta is the vector [A, B] that we are trying to optimize, and N is the number of points of vector y on the time interval ]ta; tb[ under study.

One defines D = [cos($w_j$ t[i]), sin($w_j$ t[i] ] as a matrix of size (n, 2).

The distance determination module attempts at determining y' = D * theta by minimizing the error vector e[theta] = 1 / N * (y - D * theta)$^T$ * lambda * (y - D * theta), where "$^T$" designates the transpose of a matrix.

The expression of theta which minimizes the error is theta = (D$^T$ * lambda$^{0,5}$ D)$^{-1}$ * lambda$^{0,5}$ y. In this expression "lambda$^{0,5}$" designates the vector of coordinates the square roots of the coordinates of the vector lambda.

Practically speaking, the above minimization is performed for all pulsation candidate $w_j$. The method first determines the best phase which brings the trigonometric function of pulsation $w_j$ closest to the measured signal. Then the error between the measured signal and the determined trigonometric functions is assessed for each trigonometric function, and the best trigonometric function is selected. The phase of the physiological signal at time $t_b$ is determined based on the phase of the best trigonometric function at time $t_b$. In more details, this is performed according to the following scheme:

for time tb et for a range of pulsations wj:

f. Calculation of D = [cos($w_j$ t[i]), sin($w_j$ t[i] ]
g. Calculation of PI = (D$^T$ * lambda$^{0,5}$ D)$^{-1}$ * lambda$^{0,5}$
h. Calculation of theta = PI * y
i. Calculation of the phase of y' in tb : $f_{bs}$ = -.arctan(theta[1], theta[0]), where arctan (y, x) is the angle in radians between the positive x-axis of a plane and the point given by the coordinates (*x, y*) on it, which minimizes the error of a trigonometric function y' of pulsation wj with the measured signal.

j. Calculation of an error between y' and y.

Selection, among the various functions y' of that for which the error with respect to y is minimum

The phase at time tb is the phase of the elected function y' at tb.

The phase at time tb is thus determined not taking into account any signal after tb. This allows to determine the phase in real time.

At a later time td, the process can be performed again, to determine the phase of the measured signal at td. The calculation is performed for a time interval ]tc; td[. For example, the duration of the time interval is ]tc; td[ is the same as that of ]ta; tb[. The process is repeated rapidly, so that tb will be between tc and td. For example, the process is performed at a frequency of the order of the sampling frequency of the input signal. For example, it is performed at a frequency of at least 25 Hz. For an input EEG signal, it can be performed at a frequency over 200 Hz.

According to another example, one applies a recursive least square method to determine the unknowns to be determined.

As discussed above, the expression of theta which minimizes the error is theta = $(D^T * lambda^{0,5} D)^{-1} * lambda^{0,5} y$.

A recursive expression of this problem can be written as :

Theta$_{(n+1)}$ = $(D_{(n+1)}^T * lambda^{0,5} D_{(n+1)})^{-1} * lambda^{0,5} y_{(n+1)}$, where n is an integer, n+1 is the next integer for which the problem is to be solved, $D_{(n+1)}$ is the expression of matrix D at integer (n+1), and y(n+1) is the expression of vector y at integer (n+1).

This can be written as:

$$Theta_{n+1} = (\begin{bmatrix} D_{(n)} \\ d_{n+1} \end{bmatrix}^T * lambda^{0,5} * \begin{bmatrix} D_{(n)} \\ d_{n+1} \end{bmatrix})^{-1} * \begin{bmatrix} D_{(n)} \\ d_{n+1} \end{bmatrix}^T * lambda^{0,5} * \begin{bmatrix} y_{(n)} \\ y_{n+1} \end{bmatrix},$$

By defining the matrix $S_{(n)} = (D_{(n)}^T * lambda^{0,5} D_{(n)})$, we have:

$$S_{(n+1)} = \begin{bmatrix} D_{(n)} \\ d_{n+1} \end{bmatrix}^T * lambda^{0,5} * \begin{bmatrix} D_{(n)} \\ d_{n+1} \end{bmatrix} = f(S_{(n)}).$$

In more details, this is performed according to the following scheme:

For time t et for a range of pulsations wj (in the present case, we know and use the solution to the problem at the previous time t-1):

k. Calculation of theta:

i. Calculation of $w_j.t = (2*pi*npts/(fs/f))/(2*pi)$, where fs is the signal sampling frequency and npts=t*fs, in order to adjust to the closest multiple of the signal sampling frequency,

ii. Calculation of x = [cos($w_j$ t), sin($w_j$ t]

iii. Calculation of S(t) = $lambda^{0,5}$ * S(t-1)+$x^T$.x,

iv. Calculation of gamma = S(t)$^{-1}$.$x^T$

v. Calculation of error e=y(t)-x.theta(t-1) (Alternately, if the signal is not of good enough quality, e is set equal to 0: e=0)

vi. Calculation of theta(t)=theta(t-1)+Gamma*e.

l. Calculation of the phase = -.arctan(theta[1], theta[0]), where arctan (y, x) is the angle in radians between the positive x-axis of a plane and the point given by the coordinates (*x, y*) on it, which minimizes the error of a trigonometric function y' of pulsation wj with the measured signal.

The pulsation wj which minimizes the error will be selected.

The phase at time t will be selected as the phase for the selected signal.

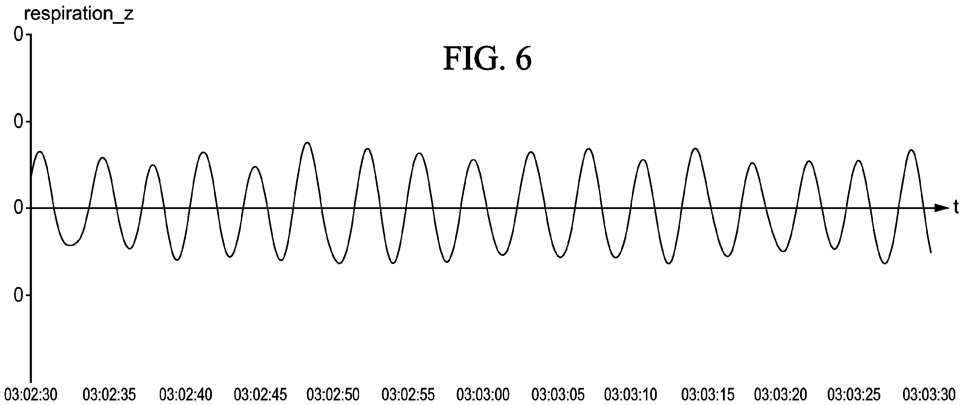

Fig. 6 shows another example of a physiological signal for which the invention can be applied. The physiological signal of Fig. 6 is a plethysmogram, i.e. a signal representative of the respiration of the user. Such as a signal may for example be obtained from a 3-axis accelerometer carried by the supporting member 2, after double integration and filtering.

In some embodiments, and for example when the signal is of poor quality, one may rely on the calculations performed for previous times when performing the calculations for the present time. This may be true notably for the respiratory signal. In particular, if the respiratory signal is obtained from acceleration measurements, the occurence of artifact movements of the person from the acceleration measurements may impair the ability to properly detect the respiratory movement. Detection, for example from the accelerometer, of such artifact movements may classify the respiratory signal as being of poor quality.

According to some embodiments, a stimulation of the user may take into account the phase of the physiological signal determined in real-time.

According to an aspect, a computer program comprises instructions to execute the steps as described above when executed on a computer.

Références :

| | | |
|---|---|---|
| device 1 for stimulating slow brain waves supporting member 2 | acoustic transducer 4 | |
| | conditioning and control electronics 5 | pulsation determination module 12 |
| arms 2a, 2b, 2c, 2d | memory 6 | phase determination module 13 |
| arm connection points 2e, 2f | communication module 7 | trigonometric function determination module 14 |
| electrodes 3 | battery 8 | |
| reference electrode 3a | adjustment device 9 | distance determination module 15 |
| EEG measurement electrode 3b | parts 9a, 9b | |
| | wire connections 10 | external server 100 |
| ground electrode 3c | signal characterization module 11 | |

**Claims**

1. Method to measure in real-time the phase of a pseudo-periodic physiological signal of a user, wherein :

   - one provides a physiological signal (S) of the user measured continuously during a time interval $]t_a\,;t_b[$ extending between times $t_a$ and $t_b$, and of duration Dt, where the physiological signal does not extend after time $t_b$ ;
   - a trigonometric function determination module (14) of a processor determines parameters of a trigonometric function, which minimize a distance between said physiological signal (S) and said trigonometric function on the time interval $]t_a\,;t_b[$, said trigonometric function being defined by at least one pulsation ($w_{bf}$) and a phase ($f_{bf}$) as said parameters ;
   - a phase determination module (13) of a processor determines the phase ($f_{ps}$) of the physiological signal at time $t_b$ based on the phase ($f_{bf}$) of the trigonometric function at time $t_b$.

2. Method according to claim 1, wherein the trigonometric function determination module (14) comprises a distance determination module (15) which affects a greater weight to points of the physiological signal closer to time $t_b$ than far away from time $t_b$.

3. Method according to claim 1 or 2, wherein the phase determination module (13) is part of a signal characterization module (11) which also comprises a pulsation determination module (12) which determines the pulsation ($w_{ps}$) of the physiological signal on time interval $]t_a\,;t_b[$ based on the pulsation ($w_{bf}$) of the trigonometric function in the time interval $]t_a\,;t_b[$.

4. Method according to any of claims 1 to 3, wherein the pseudo-periodic physiological signal has a frequency between 0.1 and 2 Hz.

5. Method according to any of claims 1 to 4, wherein the duration Dt is at least 100% of an average period of the pseudo-periodic physiological signal, at least 150% of that period, or at least 200% of that period.

6.  Method according to any of claims 1 to 5, wherein the physiological signal is an electro-encephalogram signal.

7.  Method according to any of claims 1 to 5, wherein the physiological signal is a plethysmography signal.

8.  Method according to any of claims 1 to 7, further comprising:

    - one provides a physiological signal of the user measured continuously during a time interval $]t_c ; t_d[$ extending between times $t_c$ and $t_d$, where time $t_b$ is between time $t_c$ and time $t_d$, where the physiological signal does not extend after $t_d$ ;
    - said trigonometric function determination module (14) of a processor determines the parameters of a trigonometric function, which minimize a distance between said physiological signal and said trigonometric function on the time interval $]tc ; td[$, said trigonometric function being defined by at least one pulsation ($w_{bf}$) and a phase ($f_{bf}$) ;
    - said phase determination module (13) of a processor determines the phase ($f_{ps}$) of the physiological signal at time $t_d$ based on the phase ($f_{bf}$) of the trigonometric function at time $t_d$.

9.  Method according to claim 8, wherein the time interval $]t_c ; t_d[$ is of duration Dt.

10. Method according to claim 8 or 9, wherein the phase determination module (13) is executed at a frequency at least over 25 Hz.

11. Method according to any of claims 8 to 10, wherein the phase determination module (13) of a processor determines the phase of the physiological signal at time $t_d$ based at least on the phase of the trigonometric function at time $t_b$.

12. Method according to any of claims 1 to 11, further comprising measuring said physiological signal with a sensor (3).

13. Method according to any of claims 1 to 12, further comprising emitting an acoustic stimulation signal based on the phase determined by the phase determination module (13).

14. Computer program comprising instructions adapted to perform the steps of the method of any of claims 1 to 11 wherein the computer program is run on a processor.

15. System to measure in real-time the phase of a pseudo-periodic physiological signal of a user, comprising, a physiological signal (S) of the user being provided, which was measured continuously during a time interval $]t_a ; t_b[$ extending between times $t_a$ and $t_b$, and of duration Dt, where the physiological signal does not extend after $t_b$ :

    - a trigonometric function determination module (14) of a processor adapted to determine the parameters of a trigonometric function, which minimize a distance between said physiological signal and said trigonometric function on the time interval $]t_a ; t_b[$, said trigonometric function being defined by at least one pulsation and a phase ;
    - a phase determination module (13) of a processor adapted to determine the phase of the physiological signal at time $t_b$ based on the phase of the trigonometric function at time $t_b$,

    the system optionally comprising one or more features adapted to perform the method of claims 2 to 13.

FIG. 1

FIG. 2

FIG. 3

FIG. 4

FIG. 5a

CH1 Fpz-01

FIG. 5b

EP 3 415 088 A1

respiration_z

# FIG. 6

0

0

0

t

0

0

03:02:30  03:02:35  03:02:40  03:02:45  03:02:50  03:02:55  03:03:00  03:03:05  03:03:10  03:03:15  03:03:20  03:03:25  03:03:30

Europäisches
Patentamt

European
Patent Office

Office européen
des brevets

# EUROPEAN SEARCH REPORT

Application Number

EP 17 30 5703

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/147964 A1 (COREY ROBERT A [US] ET AL) 26 May 2016 (2016-05-26)<br>* paragraphs [0110] - [0185] * | 1-15 | INV.<br>A61B5/0484<br>A61B5/00 |
| X | JACKSON JADIN C ET AL: "Computationally efficient, configurable, causal, real-time phase detection applied to local field potential oscillations",<br>2015 7TH INTERNATIONAL IEEE/EMBS CONFERENCE ON NEURAL ENGINEERING (NER), IEEE,<br>22 April 2015 (2015-04-22), pages 942-947, XP033166420,<br>DOI: 10.1109/NER.2015.7146781<br>[retrieved on 2015-07-01]<br>* abstract *<br>* /* II. Methods p. 943-945 */<br>/* IV. Discussion p. 946 */ * | 1-15 | |
| X | SANTOSTASI GIOVANNI ET AL: "Phase-locked loop for precisely timed acoustic stimulation during sleep",<br>JOURNAL OF NEUROSCIENCE METHODS,<br>vol. 259, 28 November 2015 (2015-11-28), pages 101-114, XP029381531,<br>ISSN: 0165-0270, DOI:<br>10.1016/J.JNEUMETH.2015.11.007<br>* /* 2.1 Phase-locked Loop p. 102-104 */ * | 1,3-15 | TECHNICAL FIELDS SEARCHED (IPC)<br><br>A61B |
| X | WO 2017/021661 A1 (RYTHM [FR])<br>9 February 2017 (2017-02-09)<br>* page 19, line 32 - page 22, line 25; figure 5 * | 1,3-15 | |
| A | US 2010/044551 A1 (KUSANO KOUHEI [JP])<br>25 February 2010 (2010-02-25)<br>* paragraphs [0044] - [0054] * | 1-15 | |

The present search report has been drawn up for all claims

| Place of search | Date of completion of the search | Examiner |
|---|---|---|
| Berlin | 27 November 2017 | Trachterna, Morten |

EPO FORM 1503 03.82 (P04C01)

**EP 3 415 088 A1**

## ANNEX TO THE EUROPEAN SEARCH REPORT
## ON EUROPEAN PATENT APPLICATION NO.

EP 17 30 5703

This annex lists the patent family members relating to the patent documents cited in the above-mentioned European search report.
The members are as contained in the European Patent Office EDP file on
The European Patent Office is in no way liable for these particulars which are merely given for the purpose of information.

27-11-2017

| Patent document cited in search report | | Publication date | Patent family member(s) | | Publication date |
|---|---|---|---|---|---|
| US 2016147964 | A1 | 26-05-2016 | NONE | | |
| WO 2017021661 | A1 | 09-02-2017 | FR | 3039955 A1 | 10-02-2017 |
| | | | WO | 2017021661 A1 | 09-02-2017 |
| US 2010044551 | A1 | 25-02-2010 | DE | 102009038073 A1 | 11-03-2010 |
| | | | JP | 5112989 B2 | 09-01-2013 |
| | | | JP | 2010048607 A | 04-03-2010 |
| | | | US | 2010044551 A1 | 25-02-2010 |

For more details about this annex : see Official Journal of the European Patent Office, No. 12/82